(19) Europäisches Patentamt
European Patent Office
Office européen des brevets

(11) EP 2 729 808 B1

(12) **FASCICULE DE BREVET EUROPEEN**

(45) Date de publication et mention de la délivrance du brevet:
**17.05.2017 Bulletin 2017/20**

(21) Numéro de dépôt: **12738559.9**

(22) Date de dépôt: **04.07.2012**

(51) Int Cl.:
***G01N 33/542*** *(2006.01)* ***C12Q 1/68*** *(2006.01)*

(86) Numéro de dépôt international:
**PCT/FR2012/051556**

(87) Numéro de publication internationale:
**WO 2013/004970 (10.01.2013 Gazette 2013/02)**

(54) **METHODE AMELIOREE DE DETECTION ET/OU DE QUANTIFICATION D'UN ANALYTE PRESENT A LA SURFACE D'UNE CELLULE**

VERBESSERTES VERFAHREN ZUM NACHWEIS UND/ODER ZUR QUANTIFIZIERUNG EINES ANALYTEN AN DER OBERFLÄCHE EINER ZELLE

IMPROVED METHOD FOR DETECTING AND/OR QUANTIFYING AN ANALYTE AT THE SURFACE OF A CELL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.07.2011 FR 1156103**

(43) Date de publication de la demande:
**14.05.2014 Bulletin 2014/20**

(73) Titulaire: **Cisbio Bioassays**
**30200 Codolet (FR)**

(72) Inventeurs:
- **BAZIN, Hervé**
  **30400 Villeneuve Les Avignon (FR)**
- **MATHIS, Gérard**
  **30200 Bagnols Sur Ceze (FR)**

(74) Mandataire: **Nevant, Marc et al**
**Cabinet Beau de Loménie**
**158, rue de l'Université**
**75340 Paris Cedex 07 (FR)**

(56) Documents cités:
- **N. GABORIT ET AL: "Time-resolved Fluorescence Resonance Energy Transfer (TR-FRET) to Analyze the Disruption of EGFR/HER2 Dimers: A NEW METHOD TO EVALUATE THE EFFICIENCY OF TARGETED THERAPY USING MONOCLONAL ANTIBODIES", JOURNAL OF BIOLOGICAL CHEMISTRY, vol. 286, no. 13, 1 avril 2011 (2011-04-01), pages 11337-11345, XP055021021, ISSN: 0021-9258, DOI: 10.1074/jbc.M111.223503**
- **"TR-FRET-Basics", Cisbio , 2009, XP055021123, Extrait de l'Internet: URL:http://www.htrf.com/technology/htrftheory/tr_basics/ [extrait le 2012-03-07]**
- **MAUREL D ET AL: "Cell surface detection of membrane protein interaction with homogeneous time-resolved fluorescence resonance energy transfer technology", ANALYTICAL BIOCHEMISTRY, ACADEMIC PRESS INC, NEW YORK, vol. 329, no. 2, 15 juin 2004 (2004-06-15) , pages 253-262, XP004509821, ISSN: 0003-2697, DOI: 10.1016/J.AB.2004.02.013**
- **"Homogeneous Time Resolved Fluorescence; Methodological aspects", cisbio , 2004, XP055021205, Extrait de l'Internet: URL:http://www.htrf.com/files/resources/6182008_htrf_an01.pdf [extrait le 2012-03-07]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

**(Cont. page suivante)**

- SIMONE DIERMEIER-DAUCHER ET AL: "Flow Cytometric FRET Analysis of erbB Receptor Interaction on a Cell-by-Cell Basis", ANNALS OF THE NEW YORK ACADEMY OF SCIENCES, vol. 1130, no. 1, 1 mai 2008 (2008-05-01), pages 280-286, XP55038160, ISSN: 0077-8923, DOI: 10.1196/annals.1430.003
- WATERHOUSE BENJAMIN R ET AL: "Assessment of EGFR/HER2 dimerization by FRET-FLIM utilizing Alexa-conjugated secondary antibodies in relation to targeted therapies in cancers", ONCOTARGET, vol. 2, no. 9, septembre 2011 (2011-09), pages 728-736 URL, XP002683519,

## Description

L'invention concerne une méthode améliorée de détection et / ou de quantification protéine dans un échantillon, en particulier un échantillon de tissu

## Etat de la technique

L'immunohistochimie (IHC) est le nom d'une méthode de localisation de protéines dans les cellules d'une coupe de tissu, par la détection d'antigènes au moyen d'anticorps. L'immunohistochimie exploite le fait qu'un anticorps se lie spécifiquement à des antigènes dans les tissus biologiques. Les anticorps peuvent être d'origine polyclonale ou monoclonale, les anticorps monoclonaux étant plus spécifiques par essence.

Un couple anticorps-antigène peut être visualisé de plusieurs façons. Dans la plupart des cas, un anticorps est conjugué à une enzyme (phosphatase alcaline, peroxydase de raifort notamment) générant des produits fortement colorés en présence d'un substrat chromogène (comme le DAB pour la peroxydase de raifort ?) ou bien un fluorophore (FITC, TRITC, AMCA etc.). L'analyse densitométrique du signal obtenu avec les méthodes chromogéniques ou de fluorescence peut fournir des données semi-quantitatives ou quantitatives respectivement, et permet de corréler le niveau du signal mesuré avec le niveau d'expression de protéines ou de localisation. Elle permet une détection semi-quantitative basée sur l'observation au microscope par un anatomo-pathologiste qui classe la coupe en « négative » ou « positive » (1+, 2+ ou 3+).

L'immunohistochimie est largement utilisée pour le diagnostic et/ou le suivi de cancers par détection de cellules anormales telles que celles trouvées dans les tumeurs cancéreuses. Des marqueurs spécifiques sont ainsi aujourd'hui connus pour divers cancers, tels que l'Antigène carcino-embryonnaire (CEA), utilisé en cas de cancer du côlon, CD15 et CD30, utilisés pour la maladie de Hodgkin, l'Alpha-foetoprotéine, utilisée dans la cadre de carcinomes hépatocellulaires, CD117, marqueur des tumeurs stromales gastro-intestinales et Ki-67, un des indices de prolifération tumorale. Ces marqueurs moléculaires sont caractéristiques de certains évènements cellulaires tels que la prolifération ou la mort cellulaire (apoptose).

L'immunohistochimie est aussi largement utilisée en recherche fondamentale pour comprendre la distribution et la localisation de biomarqueurs et de protéines exprimés dans les différentes parties d'un tissu biologique.

Deux approches sont utilisées en immunohistochimie : une méthode directe et une méthode indirecte. La méthode directe ne comporte qu'une seule étape de coloration et est basée sur l'utilisation d'un anticorps marqué qui réagit directement avec l'antigène dans les coupes de tissus. Bien que cette technique soit simple et rapide, sa sensibilité est faible en raison de l'absence d'amplification du signal. La méthode indirecte consiste à utiliser un anticorps primaire non marqué, spécifique de l'antigène d'intérêt, et un anticorps secondaire marqué reconnaissant les IgG de l'espèce animale dans laquelle l'anticorps primaire a été préparé. Cette méthode est plus sensible que les stratégies de détection directe en raison de l'amplification du signal due à la liaison de plusieurs anticorps secondaires à chaque anticorps primaire.

Les techniques classiques d'immunohistochimie nécessitent plusieurs étapes avant la coloration finale de l'antigène tissulaire, et de nombreux problèmes potentiels peuvent affecter le résultat de la procédure. Les problèmes les plus fréquemment rencontrés sont un bruit de fond trop important, un marquage insuffisant de l'antigène et des problèmes d'autofluorescence. Pour réduire le bruit de fond lié à la fixation non-spécifique de l'anticorps primaire ou secondaire sur des protéines présentes dans l'échantillon, ces derniers sont généralement incubés avec un tampon qui bloque les sites réactifs auxquels l'anticorps primaire ou secondaire peut se lier. Les tampons de blocage les plus fréquemment utilisé sont : le sérum normal, du lait en poudre non gras, de l'albumine sérique bovine (BSA) ou de la gélatine, et d'autres tampons aux formulations particulières sont également disponibles dans le commerce. Gaborit et al. (J. Biol. Chem. 2011, 86(13) : 11337-11345) décrit une méthode pour évaluer l'efficacité d'une thérapie ciblée utilisant des anticorps monoclonaux, méthode dans laquelle : un nombre connu de cellules sont placés dans les puits d'une plaque de microtitration et marqués à l'aide d'anticorps fluorescents ; les cellules sont incubées, marquées puis lavées ; et un signal de FRET est mesuré directement dans les puits. Les fiches techniques « TR-FRET-Basics » (URL :http://www.htrf.com/technology/htrftheory/tr_basics) et « Homogeneous Time Resolved Fluorescence ; Methodological aspects » (URL :http://www.htrf.com/files/resources/6182008_htrf an01.pdf) décrivent de manière succincte la technologie TR-FRET. Maurel et al. (Analytical Biochemistry 2004, 329(2), 253-252) décrit l'utilisation de la technologie TR-FRET pour détecter des interactions entre protéines membranaires. Diermeier-Daucher et al. (Ann. N.Y. Acad. Sci. 2008, 1130: 280-286) décrit la quantification de l'homodimérisation par la technique du FRET en flux cytométrique, en utilisant des anticorps marqués par une cyanine.

Il existe un besoin pour une technique d'analyse d'échantillons biologiques, en particulier de tissus, ne présentant pas les inconvénients des techniques classiques en termes de bruit de fond notamment.

L'invention permet de résoudre ces problèmes, en particulier elle réduit drastiquement le bruit de fond observé lorsque les approches classiques d'immunohistochimie sont mises en oeuvre, à savoir lorsqu'un seul anticorps est utilisé

pour détecter la protéine d'intérêt.

**Description**

L'invention a pour objet une méthode utilisant de manière avantageuse un couple de partenaires de FRET pour quantifier des protéines présentes à la surface d'une cellule ou dans un échantillon de tissu. L'invention à également pour objet un nécessaire de réactifs pour la mise en oeuvre de cette méthode, et l'application de cette méthode à la protéine HER2 dans le cadre d'une méthode théranostique pour déterminer si des patients souffrant de cancer du sein sont éligibles à un traitement par un anticorps anti-HER2.

Par « *couple de partenaires de FRET »* on entend un couple constitué d'un composé fluorescent donneur d'énergie (ci-après « composé fluorescent donneur ») et d'un composé accepteur d'énergie (ci-après « composé accepteur »); lorsqu'ils sont à proximité l'un de l'autre et lorsqu'ils sont excités à la longueur d'onde d'excitation du composé fluorescent donneur, ces composés émettent un signal de FRET (acronyme de l'expression anglaise « Forster Resonnance Energy Transfer »).

Par « *Signal de FRET »* on entend tout signal mesurable représentatif d'un FRET entre un composé fluorescent donneur et un composé accepteur. Un signal de FRET peut donc être une variation de l'intensité ou de la durée de vie de luminescence du composé fluorescent donneur ou du composé accepteur lorsque ce dernier est fluorescent. Lorsque le signal de FRET est mesuré en temps résolu (ce qui est en général le cas lorsque des chélates ou cryptates de terres rares sont utilisés), on parle de TR-FRET (acronyme de « time-resolved » FRET).

Un premier aspect de l'invention est relatif à une méthode de quantification d'une protéine d'intérêt présente dans un échantillon de tissu solide, comprenant les étapes suivantes :

(i) mise en contact d'un échantillon de tissu solide exprimant ladite protéine avec un premier ligand et un deuxième ligand, chacun de ces ligands étant capables de se lier de manière spécifique à un domaine de ladite protéine, et ces ligands étant respectivement marqués par un composé donneur et un composé accepteur, tous deux formant un couple de partenaires de FRET ;
(ii) incubation de l'échantillon de tissu solide avec un agent de marquage fluorescent de l'ADN ;
(iii) lavage de l'échantillon de tissu solide ;
(iv) homogénéisation de l'échantillon de tissu solide sous forme de lysat cellulaire ;
(v) mesure du signal de FRET émis par le milieu de mesure et normalisation de ce signal par le signal correspondant à la luminescence de l'agent de marquage fluorescent de l'ADN.

Par «échantillon de tissu solide », on entend un échantillon prélevé sur un patient, et de préférence un extrait de tissu tumoral.

Par « méthode de quantification » on entend une méthode de quantification relative, c'est-à-dire que le signal mesuré va être différent d'un échantillon à l'autre, en fonction de la quantité de protéine d'intérêt présente dans l'échantillon. Cette technique se distingue des méthodes classiques d'immunohistochimie dans la mesure où elle met en oeuvre deux ligands (en particulier deux anticorps) spécifiques de la protéine d'intérêt, et non pas un seul comme dans les méthodes de l'art antérieur. Elle comprend également une étape de lavage qui n'est généralement pas mise en oeuvre lorsque la technique de FRET est utilisée puisque cette approche permet des mesures en milieu homogène. Cette méthode, combinée au fait que le signal de FRET ne sera émis que lorsque ces ligands (ou anticorps) sont à proximité l'un de l'autre, résulte en un rapport signal / bruit bien meilleur que celui observé avec les protocoles classiques, à savoir ceux mettant en oeuvre un seul ligand ou anticorps.

La méthode selon l'invention nécessite des étapes de préparation de l'échantillon de tissu solide sous forme de sections de 20 à 50 μm d'épaisseur. Ces étapes de préparation sont celles qui sont classiquement mises en oeuvre dans le domaine de l'homme du métier, à savoir l'immunohistochimie. Elles peuvent consister en particulier en la fixation de l'échantillon par un traitement avec du formaldéhyde, son inclusion dans de la paraffine, en particulier sous forme de blocs qui peuvent être ensuite découpés au microtome (de préférence avec une épaisseur d'environ 20 à 50 μm). Le traitement de ces sections au xylène pour enlever la paraffine, leur rinçage à l'éthanol puis à l'eau sont également des techniques connues de l'homme du métier, de même que la régénération des épitopes par la technique HIER (acronyme de l'expression anglaise « heat induced eptitope recovery »).

De manière alternative, la méthode selon l'invention peut également être mise en oeuvre sur des cryosections, de préférence de 20 à 50 μm d'épaisseur, également préparées selon les techniques classiques.

La méthode selon l'invention comporte une étape visant à homogénéiser l'échantillon sous forme de lysat cellulaire, postérieurement à l'introduction des composés fluorescents dans le milieu de mesure (premier ligand, deuxième ligand et agent de marquage), et préalablement à la mesure du signal de FRET. Un tel traitement peut être mécanique, et peut être choisi parmi : l'application d'ultrasons (sonication), des cycles de congélation / décongélation, l'utilisation de broyeurs mécaniques, éventuellement conjointement à l'utilisation de tampon de lyse hypotonique ou de tampon de

lyse contenant des détergents comme le tampon RIPA.

Par ailleurs, il a été déterminé que les concentrations finales de premier et deuxième ligand dans le milieu de mesure sont, de manière optimale, supérieures à 10 nM, de préférence comprises entre 10 et 150 nM ou entre 20 et 80 nM, et de préférence entre 30 et 60 nM. Par concentration finale, on entend la concentration de ces composés dans le milieu de mesure une fois que tous les réactifs ont été introduits dans ce milieu. Ces domaines de concentrations sont notablement plus élevés que les concentrations habituellement utilisées dans les dosages de type TR-FRET, dans lesquels les concentrations finales de ligands fluorescents sont de l'ordre du nanomolaire, c'est à dire inférieures à 10 nM.

La méthode selon l'invention prévoit de normaliser le signal de FRET par la quantité de matériel biologique (tissu) présent dans le milieu de mesure. La méthode selon l'invention comprend ainsi l'incubation de l'échantillon de tissu avec un agent de marquage fluorescent de l'ADN (par exemple le Hoechst 33342), préalablement à l'étape de lavage, et le signal de FRET sera normalisé par le signal correspondant à la luminescence de cet agent de marquage.

Dans une mise en oeuvre préférée, le premier et le deuxième ligand sont des anticorps reconnaissant la protéine d'intérêt. Le terme « anticorps » doit ici être pris au sens large et comprends toute protéine de la famille des Immunoglobulines ou bien comportant un domaine d'une immunoglobuline, ainsi qu'un site de liaison spécifique à la protéine d'intérêt. Les anticorps peuvent donc être des fragments Fab, Fab', des anticorps simple chaîne, des domaines variables de chaînes lourdes ou légères d'immunoglobulines.

L'homme du métier est à même de fabriquer des anticorps spécifiques de la protéine dont il souhaite déterminer l'expression en utilisant les techniques classiques. De nombreux anticorps sont également disponibles dans le commerce. L'homme du métier veillera à sélectionner des anticorps reconnaissant des épitopes différents sur la protéine d'intérêt, cela pour permettre leur fixation simultanée à cette protéine.

D'autres ligands peuvent être utilisés en lieu et place des anticorps. Ainsi dans une mise en oeuvre particulière de l'invention, le premier ligand est un ligand connu (tel qu'un agoniste ou un antagoniste) de la protéine d'intérêt et n'est pas un anticorps, et le deuxième ligand est un anticorps. Là encore il est préférable que le site de liaison du premier ligand soit différent du site de liaison de l'anticorps.

Si la protéine d'intérêt est un récepteur couplé aux protéines G, l'homme du métier pourra se référer à la base de données publiée par Okuno et al. (GLIDA: GPCR ligand database for chemical genomics drug discovery database and tools update. Nucl. Acids Res., 36(suppl_1), D907-912) pour trouver des ligands utilisables dans la méthode selon l'invention.

La protéine d'intérêt peut être toute protéine exprimée par les cellules présentes dans le milieu de mesure. La méthode est particulièrement adaptée à l'étude de protéines membranaires, telles que les canaux ioniques, les récepteurs, en particulier les récepteurs couplés aux protéines G (RCPG), les récepteurs à activité tyrosine kinase (RTK), tels que par exemple : EGFR (HER1), HER2, HER3, HER4.

La méthode est particulièrement utile pour déterminer l'expression de la protéine HER2 dans des échantillons de tissus, en particulier des échantillons de tumeurs, pour déterminer si les patients concernés sont éligibles à un traitement de type anti-HER2, en particulier par un anticorps se liant à la protéine HER2 comme le trastuzumab (Herceptin™). Pour cela la valeur obtenue représentant l'expression de HER2 est comparée à une valeur seuil de référence au delà de laquelle les patients sont orientés vers une thérapie de type anti-HER2. Dans un deuxième aspect, l'invention concerne donc une méthode de sélection de patients atteints de cancers éligibles à un traitement thérapeutique par un anticorps se liant à la protéine HER2, méthode qui comprend la mise en oeuvre de la méthode de quantification selon l'invention dans laquelle protéine est HER2.

De manière tout à fait surprenante, la méthode selon l'invention a permis de détecter la protéine HER2 de manière non équivoque chez des patients « HercepTest™négatifs », à savoir chez des patients dont les résultats à ce test les rendaient inéligibles à un traitement par l'anticorps trastuzumab (patient classés 0, 1+ ou 2+). Ainsi, dans une mise en oeuvre particulièrement utile d'un point de vue clinique, la méthode selon l'invention est mise en oeuvre sur un échantillon de tissu de patient dont les résultats d'analyse par immunohistochimie de l'expression de la protéine HER2 sont négatifs (0,1+ et 2+). Cette méthode est particulièrement précieuse pour les patients ayant subi une chimiothérapie hormonale, en particulier lorsque cette dernière n'a pas entrainé d'amélioration de leur état.

Dans une mise en oeuvre préférée, la protéine d'intérêt est différente d'un récepteur exprimé de manière constitutive et exclusive sous forme d'homodimères.

Dans le cas où la protéine d'intérêt est le récepteur EGFR (HER1) l'un des ligands marqués par les partenaires de FRET peut être un anticorps anti-EGFR et l'autre ligand l'EGF ou un anticorps anti-EGFR (les anticorps anti-EGFR étant disponibles dans le commerce).

Dans le cas où la structure protéique d'intérêt est la protéine HER2, le premier et le deuxième ligands sont de préférence des anticorps spécifiques de la protéine HER2, en particulier des anticorps dont les épitopes sont situés dans le domaine extracellulaire de ce récepteur. Comme indiqué plus haut, il est préférable que ces épitopes soient différents.

**Marquages des anticorps par des composés donneurs ou accepteurs d'énergie**

Le marquage d'un ligand ou d'un anticorps par un composé donneur ou accepteur fluorescent est effectué par les techniques classiques de conjugaison faisant appel à l'utilisation de groupes réactifs. Les composés donneurs ou accepteurs fluorescents sont généralement commercialisés sous forme « fonctionnalisée », c'est-à-dire qu'ils portent un groupe réactif susceptible de réagir avec un groupe fonctionnel présent sur le composé à marquer, en l'occurrence, le ligand.

Typiquement, le groupe réactif présent sur le composé fluorescent donneur ou accepteur est un groupe électrophile ou nucléophile qui peut former une liaison covalente lorsqu'il est mis en présence d'un groupe nucléophile ou électrophile approprié, respectivement. A titre d'exemples, les couples de groupes électrophiles/nucléophiles et le type de liaison covalente formée lorsqu'ils sont mis en présence sont listés ci-dessous :

| Groupe électrophile | Groupe nucléophile | Type de liaison |
|---|---|---|
| acrylamides | thiols | thioéthers |
| halogénures d'acyle | amines/anilines | carboxamides |
| aldéhydes | amines/anilines | imines |
| aldéhydes ou cétones | hydrazines | hydrazones |
| aldéhydes ou cétones | hydroxylamines | oximes |
| alkyl sulfonates | thiols | thioéthers |
| anhydrides | amines/anilines | carboxamides |
| halogénure d'aryle | thiols | thioéthers |
| halogénure d'aryle | amines | aryl amines |
| aziridines | thiols | thioéthers |
| carbodiimides | acides carboxyliques | N-acylurées ou anhydrides |
| esters activés* | amines/anilines | carboxamides |
| haloacétamides | thiols | thioéthers |
| halotriazines | amines/anilines | aminotriazines |
| imido esters | amines/anilines | amidines |
| isocyanates | amines/anilines | urées |
| isothiocyanates | amines/anilines | thiourées |
| maléimides | thiols | thioéthers |
| sulfonate esters | amines/anilines | alkyl amines |
| halogénures de sulfonyle | amines/anilines | sulfonamides |
| * : par ester activé, on entend des groupes de formule COY, ou Y est :<br>• un groupe partant, choisi parmi les groupes succinimidyloxy ($-OC_4H_4NO_2$), sulfo succinimidyloxy ($-OC_4H_3NO_2-SO_3H$) ;<br>• un groupe aryloxy substitué ou non par au moins un substituant électrophile tel que les groupes nitro, fluoro, chloro, cyano, trifluorométhyle, formant ainsi un aryle ester activé ;<br>• un acide carboxylique activé par un groupe carbodiimide, formant un anhydride -OCORa ou -OCNRaNHRb, dans lesquels Ra et Rb sont identiques ou différents et sont choisis parmi les groupes alkyles en $C_1$-$C_6$, perfluoroalkyles en $C_1$-$C_6$, alkoxy en $C_1$-$C_6$, cyclohexyle ;<br>• le 3-diméthylaminopropyle, ou le N-morpholinoéthyle. | | |

Les composés fluorescents donneurs et accepteurs disponibles dans le commerce comportent généralement une fonction maléimide ou un ester activé, le plus souvent par un groupe NHS (N-hydroxysuccinimidyle), qui réagissent avec les groupes thiol et amines respectivement et peuvent donc être utilisés pour le marquage d'anticorps. Les anticorps marqués sont caractérisés par le rapport molaire final (RMF) qui représente le nombre moyen de molécules de marqueur

greffés au ligand.

Lorsque le ligand est de nature protéique, il peut être avantageux d'utiliser l'un des groupes fonctionnels naturellement présent dans les protéines: le groupe amino terminal, le groupe carboxylate terminal, les groupes carboxylates des acides aspartique et glutamique, les groupes amines des lysines, les groupes guanidines des arginines, les groupes thiols des cystéines, les groupes phénols des tyrosines, les cycles indoles des tryptophanes, les groupes thioéthers des méthionines, les groupes imidazoles des histidines.

Si le ligand ne comporte pas de groupe fonctionnel à l'état naturel, de tels groupes peuvent être introduits. Des méthodes d'introduction de groupes fonctionnels sont notamment décrites dans C. Kessler, Nonisotopic probing, Blotting and Sequencing, 2nd edition, L.J. Kricka (1995), Ed. Academic press Ltd., Londres, p. 66-72.

Une autre approche pour le marquage d'un ligand par un composé fluorescent consiste à introduire un groupe réactif dans le ligand, par exemple un groupe NHS ou un groupe maléimide, et de le mettre en présence d'un fluorophore portant un groupe fonctionnel qui réagira avec le groupe réactif pour former une liaison covalente.

Il est important de vérifier que le ligand marqué conserve une affinité suffisante pour son récepteur ; cela peut être contrôlé de manière simple par des expériences de liaison classiques, permettant de calculer la constante d'affinité du ligand marqué pour le récepteur.

## Couples de partenaires de FRET

Les couples de partenaires de FRET sont de préférence constitués par un composé fluorescent donneur et un composé fluorescent accepteur d'énergie.

Le FRET est défini comme un transfert d'énergie non radiatif résultant d'une interaction dipôle-dipôle entre un donneur et un accepteur d'énergie. Ce phénomène physique nécessite une compatibilité énergétique entre ces molécules. Cela signifie que le spectre d'émission du donneur doit recouvrir, au moins partiellement, le spectre d'absorption de l'accepteur. En accord avec la théorie de Förster, le FRET est un processus qui dépend de la distance séparant les deux molécules, donneur et accepteur : lorsque ces molécules sont à proximité l'une de l'autre, un signal de FRET sera émis.

La sélection du couple fluorophore donneur / accepteur pour obtenir un signal de FRET est à la portée de l'homme du métier. Des couples donneur-accepteur utilisables pour étudier les phénomènes de FRET sont notamment décrits dans l'ouvrage de Joseph R. Lakowicz (Principles of fluorescence spectroscopy, 2nd edition, Kluwer academic/plenum publishers, NY (1999)), auquel l'homme du métier pourra se référer.

Les composés fluorescents donneurs d'énergie à durée de vie longue (> 0,1 ms, de préférence comprise entre 0,5 et 6 ms), en particulier les chélates ou cryptates de terres rares sont avantageux puisqu'ils permettent d'effectuer des mesures en temps résolu, c'est-à-dire de mesurer des signaux de TR-FRET (en langue anglaise, « Time Resolved FRET ») en s'affranchissant du phénomène d'autofluorescence émis par le milieu de mesure. Ils sont pour cette raison et de manière générale préférés pour la mise en oeuvre de la méthode selon l'invention.

Les complexes de dysprosium (Dy3+), de samarium (Sm3+), de neodyme (Nd3+), d'ytterbium (Yb3+) ou encore d'erbium (Er3+) sont des complexes de terres rares également appropriés aux fins de l'invention, mais les chélates et cryptates d'europium (Eu3+) et de terbium (Tb3+) sont particulièrement préférés.

De très nombreux complexes de terres rares ont été décrits et plusieurs sont actuellement commercialisés par les sociétés PerkinElmer, Invitrogen et Cisbio Bioassays.

Des exemples de chélates ou cryptates de terres rares appropriés aux fins de l'invention sont :

- Les cryptates de lanthanides, comportant un ou plusieurs motifs pyridine. De tels cryptates de terre rare sont décrits par exemple dans les brevets EP 0 180 492, EP 0 321 353, EP 0 601 113 et dans la demande internationale WO 01/96 877. Les cryptates de terbium (Tb3+) et d'europium (Eu3+) sont particulièrement appropriés aux fins de la présente invention. Des cryptates de lanthanides sont commercialisés par la société Cisbio Bioassays. On peut citer à titre d'exemple non limitatif les cryptates d'europium de formules ci-dessous (qui peuvent être couplés au composé à marquer via un groupe réactif, ici par exemple un groupe $NH_2$) :

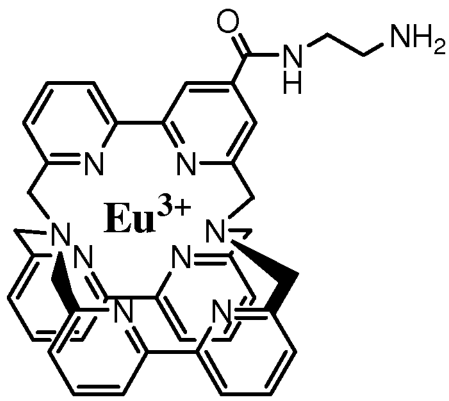

**TrisBiPy-Eu**

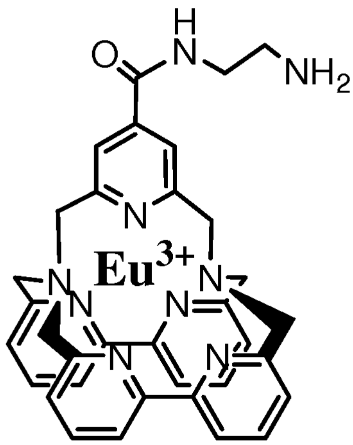

**Py-BiPy-Eu**

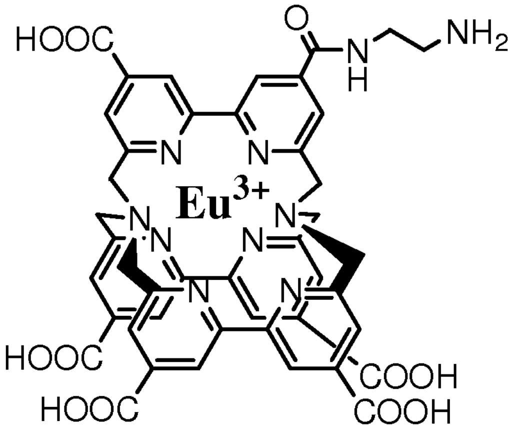

**TrisBiPy-tetraacide-Eu**

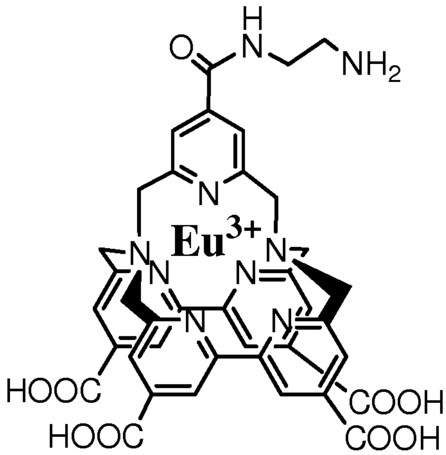

**Py-BiPy-tetraacide-Eu**

- Les chélates de lanthanides décrits notamment dans les brevets US 4 761 481, US 5 032 677, US 5 055 578, US 5 106 957, US 5 116 989, US 4 761 481, US 4 801 722, US 4 794 191, US 4 637 988, US 4 670 572, US 4 837 169, US 4 859 777. Les brevets EP 0 403 593, US 5 324 825, US 5 202 423, US 5 316 909 décrivent des chélates composés d'un ligand nonadenté tel que la terpyridine. Des chélates de lanthanides sont commercialisés par la société PerkinElmer.
- Des complexes de lanthanides constitués d'un agent chélatant, tel que le tétraazacyclododécane, substitué par un chromophore comportant des cycles aromatiques, tels que ceux décrits par Poole R. et al. dans Biomol. Chem, 2005, 3, 1013-1024 "Synthesis and characterisation of highly emissive and kinetically stable lanthanide complexes suitable for usage in cellulo", peuvent également être utilisés. On peut aussi utiliser les complexes décrits dans la demande WO 2009/10580.
- Les cryptates de lanthanides décrits dans les brevets EP 1 154 991 et EP 1 154 990 sont également utilisables.
- Le cryptate de terbium de formule ci-dessous (qui peut être couplé à un composé à marquer via un groupe réactif, ici par exemple un groupe NH2) :

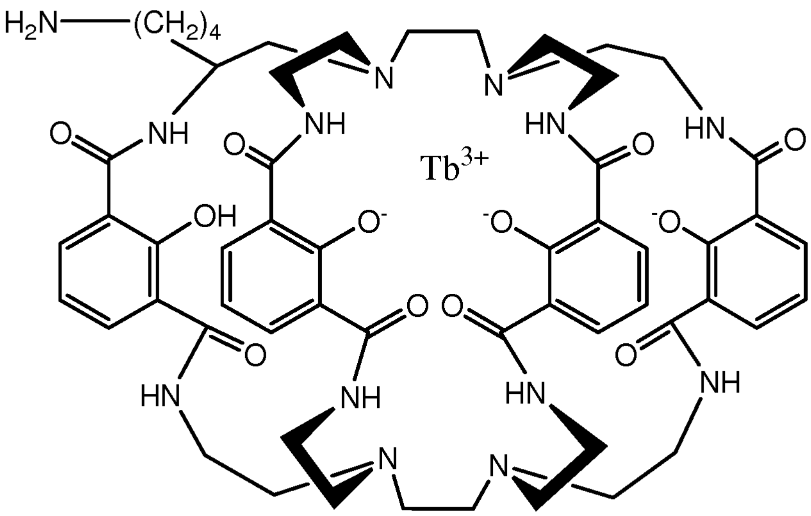

et dont la synthèse est décrite dans la demande internationale WO 2008/063721 (composé 6a page 89).

- Le cryptate de terbium Lumi4-Tb de la société Lumiphore, commercialisé par Cisbio Bioassays.
- Le quantum dye de la société Research Organics, de formule ci-dessous (qui peut être couplé au composé à marquer via un groupe réactif, ici NCS) :

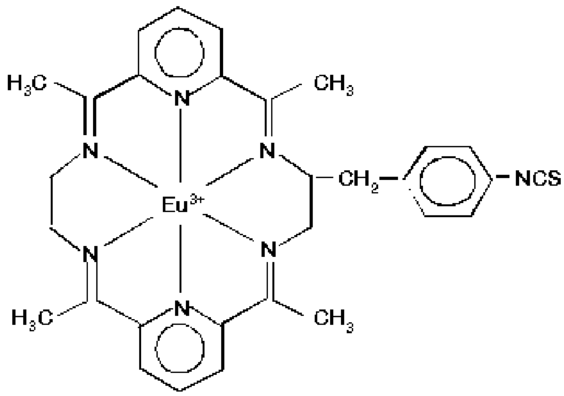

Les chélates de ruthénium, en particulier les complexes constitués d'un ion ruthénium et de plusieurs bipyridines comme le ruthénium(II) tris(2,2'-bipyridine).

Le chélate de terbium DTPA-cs124 Tb, commercialisé par la société Life technologies de formule ci-dessous (qui peut être couplé au composé à marquer via un groupe réactif R) et dont la synthèse est décrite dans le brevet américain US 5 622 821.

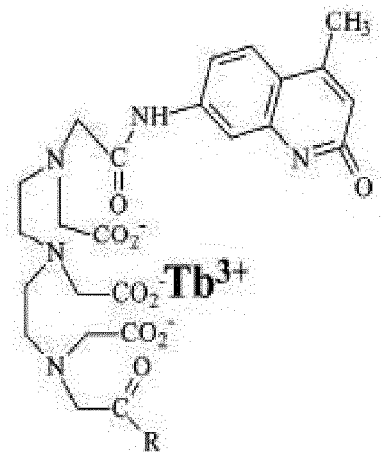

Le chélate de terbium de formule ci-dessous et décrit par Latva et al (Journal of Luminescence 1997, 75: 149-169) :

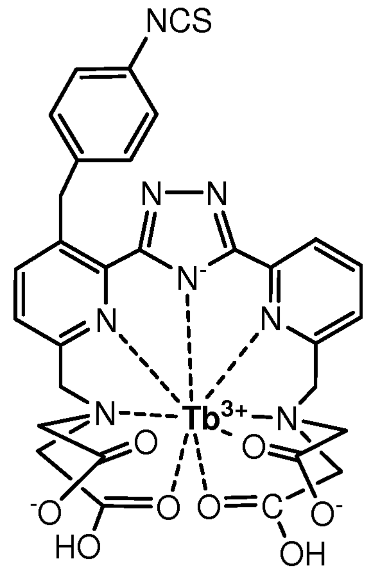

Tb-W14016

De manière particulièrement avantageuse, le composé fluorescent donneur est choisi parmi : un cryptate d'europium; un chélate d'europium ; un chélate de terbium ; un cryptate de terbium ; un chélate de ruthénium ; et un quantum dye ; les chélates et les cryptates d'europium et de terbium étant particulièrement préférés.

Les complexes de dysprosium (Dy3+), de samarium (Sm3+), de néodyme (Nd3+), d'ytterbium (Yb3+) ou encore d'erbium (Er3+) sont également des complexes de terres rares appropriés aux fins de l'invention.

Les composés fluorescents accepteurs peuvent être choisis dans le groupe suivant : les allophycocyanines, en particulier celles connues sous la dénomination commerciale XL665 ; les molécules organiques luminescentes, telles que les rhodamines, les cyanines (comme par exemple Cy5), les squaraines, les coumarines, les proflavines, les acridines, les fluorescéines, les dérivés du boron-dipyrrométhène (commercialisés sous la dénomination « Bodipy »), les fluorophores connus sous la dénomination « Atto », les fluorophores connus sous la dénomination «DY», les composés connus sous la dénomination « Alexa », le nitrobenzoxadiazole. Avantageusement, les composés fluorescents accepteurs sont choisis parmi les allophycocyanines, les rhodamines, les cyanines, les squaraines, les coumarines, les proflavines, les acridines, les fluorescéines, les dérivés du boron-dipyrrométhène, le nitrobenzoxadiazole.

Les expressions « les cyanines » et « les rhodamines » doivent être respectivement comprises comme « les dérivés de cyanine » et « les dérivés de rhodamine ». L'homme du métier connaît ces différents fluorophores, disponibles

dans le commerce.

Les composés « Alexa » sont commercialisés par la société Invitrogen; les composés « Atto » sont commercialisés par la société Atto-tec ; les composés « DY» sont commercialisés par la société Dyomics ; les composés « Cy » sont commercialisés par la société Amersham Biosciences ; les autres composés sont commercialisés par divers fournisseurs de réactifs chimiques, tels que les sociétés Sigma, Aldrich ou Acros.

Les protéines fluorescentes suivantes peuvent également être utilisées en tant que composé fluorescent accepteur : les protéines fluorescentes cyans (AmCyan1, Midori-Ishi Cyan, mTFP1), les protéines fluorescentes vertes (EGFP, AcGFP, TurboGFP, Emerald, Azami Green, ZsGreen), les protéines fluorescentes jaunes (EYFP, Topaz, Venus, mCitrine, YPet, PhiYFP, ZsYellow1, mBanana), les protéines fluorescentes oranges et rouges (Orange kusibari, mOrange, tdtomato, DsRed, DsRed2, DsRed-Express, DsRed-Monomer, mTangerine, AsRed2, mRFP1, JRed, mCherry, mStrawberry, HcRed1, mRaspberry, HcRed-Tandem, mPlim, AQ143), les protéines fluorescentes dans le rouge lointain (mKate, mKate2, tdKatushka2).

Aux fins de l'invention, les dérivés de cyanines ou de la fluorésceine sont préférés comme composés fluorescents accepteurs.

## Exemples

### Exemple 1

Des cellules NIH/3T3 (fibroblastes de souris n'exprimant pas le récepteur EGFR humain) ont été transfectées de manière stable à l'aide d'un plasmide contenant la séquence codant pour hEGFR, et sélectionnées dans un milieu contenant de la puromycine. La lignée obtenue, exprimant le récepteur hEGFR, sera par la suite nommée **P1.** De manière similaire (à l'exception du milieu de sélection qui contenait de l'hygromycine), une lignée exprimant la protéine HER2 humain a été préparée (ci-après lignée « **H2** »).

La tumeur obtenue par xenogreffes de cellules P1 sur une souris et fixée dans le formaldéhyde selon un protocole classiquement utilisé a été ensuite incluse dans de la paraffine. Les blocs ainsi obtenus ont été coupée au microtome (épaisseur ~ 20 μm) et les sections FFPE (acronyme de « Formaldehyde Fixed Paraffin Embedded ») ainsi obtenues ont été conservées dans un tube type eppendorff à 4°C jusqu'à utilisation.

A une section contenue dans un tube eppendorf ont été ajoutés 1,2 mL de substitut de Xylène et après 5 min d'incubation, le tube a été centrifugé (16 000 RCF) et le surnageant éliminé par pipetage. Le même processus a été renouvelé une fois, puis 1,2 mL d'Ethanol absolu ont été ajoutés. Après 3 min d'incubation le tube a été centrifugé (16 000 RCF) et le surnageant éliminé par pipetage. Le même processus a été renouvelé une fois, puis 1,2 mL d'Ethanol à 95% ont été ajoutés. Après 3 min d'incubation le tube a été centrifugé (16 000 RCF) et le surnageant éliminé par pipetage. Ensuite, 1,2 mL d'Ethanol à 50% ont été ajoutés, et après 3 min d'incubation le tube a été centrifugé (16 000 RCF) et le surnageant éliminé par pipetage. 0,5 mL de tampon 10 mM TRIS-HCl + EDTA (pH9) ont été ajoutés puis le tube fermé et chauffé au bain marie pendant 20 min. Après refroidissement (10 min) le tube a été centrifugé (16 000 RCF) et le surnageant éliminé par pipetage.

La section dont les épitopes d'EGFR ont été régénérés par traitement thermique (en Anglais : HIER pour Heat Induced Epitope Recovery) a été remise en suspension dans un tampon d'incubation constitué de 180 μL de tampon HEPES 50 mM pH7 contenant un cocktail d'inhibiteurs de protéases, 1 % de BSA, et une concentration finale de 50 nM d'un anticorps Ab15 spécifique d'EGFR (Thermo Fisher) marqué à l'aide de cryptate Lumi4® Tb (Ab15-Lumi4Tb®, Cisbio Bioassays) et de 50 nM d'un anticorps REGF01 également spécifique de EGFR (Cisbio Bioassays) et marqué à l'aide du fluorophore accepteur d2 (Cisbio Bioassays), qui émets à 665 nm (REGF01-d2).

Après 16h d'incubation à 20°C, 20 μL d'une solution de Hoechst 33342 à 20 μg/ mL dans du tampon ont été ajoutés, puis après 15 min d'incubation, le tube a été centrifugé (16 000 RCF), le surnageant éliminé par pipetage. Le culot a été remis en suspension dans 300 μL de tampon HEPES 50 mM contenant un cocktail d'inhibiteurs de protéases et 0,1 % de BSA, et le tube a été centrifugé (16 000 RCF). Le cycle de lavage/centrifugation dans le même tampon a été répété deux fois, et les surnageant éliminés. Après sonication (5s à 20% d'intensité sur sonicateur Branson) permettant une homogénéisation, 100 μL du lysat obtenu ont été pipetés dans les puits B3 et B4 d'une microplaque noire (96 puits).

Un échantillon de « contrôle négatif » (n'exprimant pas le récepteur EGFR) a été préparé de manière similaire mais à partir d'une tumeur elle-même obtenue par xenogreffes de cellules H2 sur une souris. 100 μL du lysat obtenu ont été pipetés dans chacun des puits B1 et B2 de la microplaque noire de façon à constituer un témoin négatif.

Dans les puits adjacents ont été déposés également du tampon seul (BT : blanc tampon) et 100 μL de solutions diluées du mélange d'anticorps REGF01-d2 Ab15-Lumi4Tb® (tampon d'incubation) obtenus par dilution en cascade au ½ (dans le tampon HEPES 0,1% BSA) de façon à obtenir une gamme de concentration finale en anticorps marqués allant de 0,16 nM à 5 nM (puits A3 à A12). On a ensuite mesuré successivement en mode fluorescence (« prompt fluorescence » délai = 0 ; durée de mesure après délai = 20 μs) sur un appareil TECAN Saphir 2 en excitant l'accepteur

(d2) à 640 nm et en lisant la fluorescence émise à 665 nm, puis la fluorescence en mode temps résolu simultanément à 620 nm (Lumi4Tb®) et 665 nm (délai = 60 $\mu$s ; durée de la mesure après délai = 400 $\mu$s) sur un appareil Pherastar (excitation par lampe flash) après excitation à 340 nm.

Les mesures en mode fluorescence à 665 nm (F665) des puits de la gamme (0,16 nM à 5 nM) permettent d'obtenir une droite étalon reliant la fluorescence corrigée pour chaque puits par soustraction de la fluorescence du tampon seul (B665) :

F665c = F665-B665 (voir figure 1).

Par interpolation on obtient la concentration en anticorps liés au matériel biologique.

Tableau 1

| | Cellules H2 (pas d'hEGFR) | | Cellules P1 (hEGFR) | |
|---|---|---|---|---|
| **F665c** | 28 070 | 28 080 | 45 370 | 45 200 |
| **Ac-d2 (nM)** | 2,8 | 2,8 | 4,6 | 4,6 |

Le rapport du signal spécifique (fixation REGF01-d2 sur EGFR) sur le signal du contrôle négatif est de 4,6 / 2,8 = 1,64, ce qui est un rapport signal/bruit relativement faible.

De la même façon les mesures en mode TRF à 620 nm des puits de la gamme (0,16 nM à 5 nM) permettent d'obtenir une droite étalon reliant la fluorescence corrigée pour chaque puits par soustraction de la fluorescence du tampon seul (B620) :

E620c = E620-B620 (voir figure 2).

Tableau 2

| | Cellules H2 (pas d'hEGFR) | | Cellules P1 (hEGFR) | |
|---|---|---|---|---|
| **E620c** | 33 930 | 33 300 | 115 500 | 114 100 |
| **AcLumi4 Tb (nM)** | 0,80 | 0,79 | 2,73 | 2,70 |

Le rapport du signal spécifique (fixation Ab15-Lumi4Tb® sur EGFR) sur le signal du contrôle négatif est de 2,72 / 0,8 = 3,4, ce qui est également relativement mauvais.

On mesure la plaque en mode TRF simultanément à 620 nm (luminescence du donneur Lumi4Tb®) et 665 nm (luminescence de l'accepteur d2).

Pour la gamme de calibration on obtient les valeurs suivantes, reportées dans le tableau3.

Tableau 3

| **Ac Lumi4 Tb (nM)** | **0,16** | **0,31** | **0,63** | **1,25** | **2,5** | **5** |
|---|---|---|---|---|---|---|
| E620 | 6 650 | 13 200 | 27 270 | 53 600 | 106 450 | 210 700 |
| E665 | 609 | 1 150 | 2 360 | 4 630 | 9 260 | 18 250 |

On trace le graphe représentant E665 = f(E620), et on obtient une droite dont la pente permet de calculer la contribution de l'émission résiduelle du terbium à 665 nm à partir du signal mesuré à 620 nm pour les échantillons H2 et P1 qui est nommé B665 dans le tableau suivant.

On calcule le signal de FRET restitué à 665 nm en calculant la différence entre le signal à 665 nm (E665c) et l'émission résiduelle du terbium dans le canal 665 nm : $\Delta$665 = E665c - B665.

Dans le tableau ci-dessous H460c correspond à la fluorescence à 460nm du composé Hoechst 33342, mesurée en mode fluorescence classique.

Tableau 4

| | Cellules H2 (pas d'hEGFR) | | Cellules P1 (hEGFR) | |
|---|---|---|---|---|
| **E665** | 4040 | 4060 | 169 700 | 164 200 |
| **E665c** | 3 875 | 3 900 | 169 560 | 164 060 |
| **B665** | 2 920 | 2 860 | 9 940 | 9 8100 |
| **∆665** | 960 | 1 035 | 159 630 | 154 250 |
| **H460c** | 32 300 | 34 100 | 57 630 | 60 270 |
| **∆665N** | 297 | 303 | 27 699 | 25 593 |
| **∆665N moyen** | 300 | | 26 646 | |

Le rapport du signal spécifique obtenu selon l'invention (fixation REGF01-d2 et Ab15-Lumi4Tb® sur EGFR) sur le signal du contrôle négatif est de 26646 / 300 = 89, ce qui est excellent.

Cet exemple montre que la méthode selon l'invention permet d'obtenir un excellent rapport signal / bruit (facteur 89) alors que l'utilisation d'un seul anticorps marqué ne permets d'obtenir qu'un facteur de 3,42, au mieux, entre le signal spécifique et le bruit de fond.

## Exemple 2

Dans les puits d'une plaque de microtitration (plaque noire 96 puits fond plat) des cellules de la lignée A431 (ATCC/CRL-1555 exprimant environ $2.10^6$ récepteurs EGFR par cellule (Shinobu, 1984 Molecular and Cellular Endocrinology 37, 205) ont été distribuées à une densité de 25 000 cellules par puits et incubées une nuit à 37 °C en milieu DMEM de façon à obtenir un tapis de cellules adhérentes.

Après deux lavages par 100 µL de PBS, 50 µL de tampon KREBS ont été ajoutés par puits.

[Tampon Krebs/HEPES mmol/L: NaCl 99 ; KCl 4,69 ; $CaCl_2$ 1,87 ; $MgSO_4$ 1,2 ; $K_2HPO4$ 1,03 ; $NaHCO_3$ 25 ; Na-HEPES 20 + glucose 11,1 mM + 0,1% BSA pH 7,4].

Dans les puits A1 à A4 aucun autre réactif n'a été ajouté (blanc tampon). Dans les puits A9 à A12 ont été ajoutés 10 µL d'EGF 1µM dans du KREBS (pour saturer les sites de liaison à l'EGF), puis dans les puits A5 à A12 un mélange d'anticorps Ab10-d2 (anti-EGFR Ab10 Thermo Fisher Scientific marqué par du d2-NHS Cisbio Bioassays à un RMF = 1,8) et d'EGF-Lumi4 Tb (Cisbio Bioassays). Le volume de chaque puits a été complété à 100 µL par du tampon KREBS de façon à obtenir une concentration finale dans les puits de 5 nM en Ab10-d2 et 5 nM en EGF-Lumi4 Tb.

Dans des puits de la même plaque de microtitration, une gamme étalon a été constituée par dilution dans du tampon KREBS d'une solution mère contenant EGF-Lumi4 Tb et Ab10-d2 (dilution en cascade au ½ de façon à obtenir des concentrations finales en Ac de 1 nM à 0,031 nM). Une lecture en mode temps résolu a été effectuée sur un appareil Pherastar FS (BMG) en utilisant les paramètres suivants :

| | |
|---|---|
| Nombre de flashes par puits | 300 |
| Module optique: | HTRF |
| Excitation (nm) | 337 |
| Emission A (nm) | 665 |
| Emission B (nm) | 620 |
| Début de l'Intégration [µs]: | 60 |
| Durée de l'Intégration [µs]: | 400 |
| Hauteur de focalisation [mm]: | 3.9 |
| Ratio multiplicateur: | 10 000 |
| Source Lumineuse: | lampe flash |

Les valeurs d'émission E620c obtenues (après soustraction de la valeur du blanc B620 mesuré sur les puits A1 et A2 contenant uniquement du tampon) ont été reportées dans le tableau suivant.

Tableau 5

| **EGF-Lumi 4 Tb (nM)** | 0,031 | 0,063 | 0,125 | 0,25 | 0,5 | 1 |
|---|---|---|---|---|---|---|

(suite)

| | | | | | | |
|---|---|---|---|---|---|---|
| **E620c** | 634 | 930 | 1 490 | 3 050 | 5 500 | 10 850 |

Le graphe E620c = f (EGF-Lumi4Tb) a permis d'obtenir la correspondance entre E620c et la concentration en nM de l'EGF-Lumi4Tb (voir figure 3).

La plaque a été incubée 4 heures à 20°C (à l'obscurité), avant l'ajout de 10 μL de Hoechst 33342 (solution 2mg/ml en DMSO prédiluée extemporanément au 1/100$^{ème}$ dans du KREBS) dans les puits A3 à A12 et incubation pendant 1 heure supplémentaire à 20°C. Tous les puits ont alors été lavés par 4 fois 100 μL de tampon KREBS et 100 μL du même tampon ajouté dans chaque puits. Une lecture de fluorescence en temps résolu a été effectuée avec les mêmes paramètres que ci-dessus.

Tableau 6

| **Puits** | **5** | **6** | **7** | **8** | **9** | **10** | **11** | **12** |
|---|---|---|---|---|---|---|---|---|
| | **Pas d'EGF ajouté** | | | | **+ EGF (100nM final)** | | | |
| E620c | 3 870 | 4 710 | 3 390 | 3 280 | 1 180 | 1 340 | 770 | 910 |
| **E620c moyen** | 3810 | | | | 1 050 | | | |
| **EGF-Lumi4Tb (nM)** | 0,348 | | | | 0,096 | | | |

Les puits dans lesquels de l'EGF (100 nM final) a été ajouté pour saturer les sites de liaison des récepteurs EGFR exprimés par les cellules (puits A9 à A12), ont permis d'obtenir une valeur de fluorescence E620c (moyenne = 1050 UFA) qui représente le bruit de fond correspondant à la fixation non spécifique de l'EGF marqué au Lumi4 Tb sur les cellules. Grâce à la droite d'étalonnage il a pu être évalué que la fixation d'EGF-Lumi4 Tb est de l'ordre de 0,096 nM. La fixation spécifique d'EGF-Lumi4 Tb sur les cellules (puits A5 à A8) est de l'ordre de 0,348 nM. Le rapport signal/bruit lorsque un seul ligand du récepteur d'intérêt est utilisé (EGF-Lumi4Tb) est donc de 3,6 ce qui est relativement faible.

Pour tenir compte de la variabilité du nombre de cellules dans les puits (due notamment au décollement des cellules suite aux lavages) une normalisation utilisant le signal du Hoechst 33342 a été effectuée en divisant le signal E620c par la valeur de fluorescence à 460nm mesurée en mode fluorescence « classique » pour le complexe Hoechst/ADN dans chaque puits (et en multipliant par 100 000 pour obtenir des nombres entiers).

Tableau 7

| | | | | | | | | |
|---|---|---|---|---|---|---|---|---|
| **H460c** | 67 380 | 99 330 | 79 590 | 106 300 | 82 990 | 109 800 | 74 400 | 88 000 |
| **E620N** | 5 750 | 4 740 | 4 250 | 3 090 | 1 420 | 1 220 | 1 040 | 1 040 |
| **E620N moyen** | 4 460 | | | | 1 180 | | | |

Le rapport signal/bruit est de 3,78 après normalisation des valeurs par le signal du Hoechst 33342, ce qui est également relativement faible.

**Mesure de la fixation de l'Anticorps marqué (Ab10-d2):**

D'après la fiche technique du fabriquant, l'Ab10 se fixe sur un épitope proche du site de liaison de l'EGFR car cet anticorps perturbe la liaison de l'EGF sur l'EGFR. L'ajout d'EGF en excès doit également perturber la fixation de l'anticorps ce qui permet d'estimer la liaison non spécifique de cet anticorps en ajoutant un excès d'EGF.

Des mesure de fluorescence ont été effectuées en mode fluorescence en excitant l'accepteur à 640 nm et en mesurant son émission (« prompt fluorescence ») à 680 nm (compte-tenu de la largeur de bande des filtres on est en droit d'assimiler les mesures faites avec un filtre « 665 nm » et « 680 nm » comme étant, dans les deux cas, une mesure correspondant au canal « accepteur »)

| | |
|---|---|
| No. de flashes par puits | 100 |
| Module Optique: | FI 640 680 |
| Excitation (nm) | 640 |

(suite)

| | |
|---|---|
| Emission (nm) | 680 |
| Gain | 2459 |

Les mesures E680c après soustraction du blanc tampon mesuré à 680 nm ont permis d'obtenir les concentrations en nM d'anticorps fixé sur les cellules en utilisant une gamme de calibration d'Ab10-d2 (1nM à 0,031 nM).

Tableau 8

| **3** | **4** | **5** | **6** | **7** | **8** | **9** | **10** | **11** | **12** |
|---|---|---|---|---|---|---|---|---|---|
| | | **pas d'EGF ajouté** | | | | **+EGF (100nM final)** | | | |
| E680c | | 102 800 | 105 900 | 71 500 | 91 500 | 53 800 | 65 350 | 54 750 | 62 800 |
| **E680c moyen** | | 92 950 | | | | 59 180 | | | |
| **Ab10-d2 (nM)** | | 6,74 | | | | 4,29 | | | |

Le rapport signal/bruit lorsque un seul ligand du récepteur d'intérêt est utilisé (Ab10-d2) est donc de 1,58, ce qui est relativement faible

**Mesure de la fixation des sondes fluorescentes par TR-FRET :**

De façon analogue, les signaux à 620 nm et à 665nm ont été mesurés en mode temps résolu dans les puits contenant des dilutions d'EGF-Lumi4 Tb et d'Ab10-d2 (1nM à 0,031 nM).

Tableau 9

| | | | | | | |
|---|---|---|---|---|---|---|
| **E620c** | 634 | 930 | 1 490 | 3 050 | 5 500 | 10 850 |
| **E665c** | 13 | 43 | 106 | 220 | 466 | 1 210 |

La droite de corrélation entre E665c et E620c permet d'obtenir la contribution de l'émission du terbium dans le canal 665nm : E665 = 0.102 x E620, ce qui permet d'obtenir les valeurs B665 correspondant à la contribution de l'émission du terbium.

Les résultats des mesures à 665nm en temps résolu (paramètres ci dessus) ont été rassemblés dans le tableau suivant, dans lequel Δ665 = E665c - B665.

Tableau 10

| | **Pas d'EGF ajouté** | | | | **+ EGF (100nM final)** | | | |
|---|---|---|---|---|---|---|---|---|
| **Puits** | **5** | **6** | **7** | **8** | **9** | **10** | **11** | **12** |
| **E665c** | 2060 | 2 390 | 1 600 | 1 860 | 390 | 480 | 290 | 320 |
| **B665** | 395 | 480 | 345 | 335 | 120 | 137 | 79 | 93 |
| **Δ665** | 1 660 | 1 910 | 1 260 | 1 520 | 270 | 340 | 215 | 223 |
| **Δ665 moyen** | 1 588 | | | | 262 | | | |

Dans ce cas le signal obtenu à 665nm représente le signal émis par l'accepteur (Ab10-d2) excité par FRET avec le donneur (EGF-Lumi4 Tb).

Le rapport signal/bruit lorsque la méthode selon l'invention est mise en oeuvre, c'est-à-dire en utilisant deux ligands marqués par des partenaires de FRET, est de 6,0, ce qui plus élevé que ce qui est observé avec le ligand marqué seul ou l'anticorps marqué seul.

Pour tenir compte de la variabilité du nombre de cellules dans les puits, une normalisation utilisant le signal du Hoechst 33342 a été effectuée en divisant le signal Δ665 par la valeur de fluorescence mesurée à 460nm en mode fluorescence « classique » pour le complexe Hoechst/ADN dans chaque puits (et en multipliant par 100 000 pour obtenir des nombres entiers).

Tableau 11

| | Pas d'EGF ajouté | | | | + EGF (100nM final) | | | |
|---|---|---|---|---|---|---|---|---|
| **Puits** | **5** | **6** | **7** | **8** | **9** | **10** | **11** | **12** |
| **Δ665** | 1 660 | 1 910 | 1 260 | 1 520 | 270 | 340 | 215 | 223 |
| **H460c** | 67 380 | 99 330 | 79 590 | 106 300 | 82 990 | 109 800 | 74 400 | 88 000 |
| **Δ665N** | 2463 | 1923 | 1583 | 1430 | 325 | 309 | 289 | 253 |
| **Δ665N moyen** | 1 849 | | | | 294 | | | |

Le rapport signal/bruit est alors de 6,29, donc plus élevé que ce qui est observé avec le ligand marqué seul ou l'anticorps marqué seul.

## Exemple 3

Dans les puits d'une plaque de microtitration (plaque noire 96 puits fond plat) des cellules de la lignée A431 (ATCC/CRL-1555 exprimant environ $2.10^6$ récepteurs EGFR par cellule (Shinobu, 1984 Molecular and Cellular Endocrinology 37, 205) ont été distribuées à une densité de 25 000 cellules par puits et incubées une nuit à 37 °C en milieu DMEM de façon à obtenir un tapis de cellules adhérentes.

On a lavé par 2x 100 μL PBS puis ajouté 50 μL de tampon KREBS par puits. [Tampon Krebs/HEPES mmol/L: NaCl 99 ; KCl 4,69 ; $CaCl_2$ 1,87 ; $MgSO_4$ 1,2 ; $K_2HPO4$ 1,03 ; $NaHCO_3$ 25 ; Na-HEPES 20 + glucose 11,1 mM + 0,1% BSA pH 7,4].

Dans les puits B1 à B4 aucun autre réactif n'a été ajouté (blanc tampon). Dans les puits B5 à B8 a été ajouté un mélange d'anticorps Ab10-d2 (anti-EGFR Ab10 Thermo Fisher Scientific marqué par du d2-NHS Cisbio Bioassays) et du Cetuximab-Lumi4 Tb (anti-EGFR) et le volume complété à 100 μL par du tampon KREBS de façon à obtenir une concentration finale dans les puits de 5 nM en Ab10-d2 et 5 nM en Cetuximab-Lumi4 Tb.

Dans des puits de la même plaque de microtitration, une gamme étalon a été constituée par dilution dans du tampon KREBS d'une solution mère contenant EGF-Lumi4 Tb et Ab10-d2 (dilution en cascade au ½ de façon à obtenir des concentrations finales en Ac de 1 nM à 0,031 nM). Une lecture en mode temps résolu a été effectuée sur un appareil Pherastar FS (BMG) en utilisant les paramètres suivants :

| | |
|---|---|
| Nombre de flashes par puits | 300 |
| Module optique: | HTRF |
| Excitation (nm) | 337 |
| Emission A (nm) | 665 |
| Emission B (nm) | 620 |
| Début de l'Intégration [μs]: | 60 |
| Durée de l'Intégration [μs]: | 400 |
| Hauteur de focalisation [mm]: | 3,9 |
| Ratio multiplicateur: | 10 000 |
| Source Lumineuse: | lampe flash |

Pour évaluer la fixation non spécifique des anticorps à la surface des cellules, des cellules CHO (Chines Hamster Ovary) ont été incubées avec la même solution d'anticorps marqués, les puits ont été lavés et la fluorescence mesurée dans les mêmes conditions.

Après lavage, le signal moyen émis par Cetuximab-Lumi4 Tb seul est de 24 033 UAF (unités arbitraires de fluorescence) pour les cellules A431 et de 1 170 UFA pour les cellules CHO, soit un rapport signal/bruit de 21.

Lorsque la méthode selon l'invention est mise en oeuvre et que l'on mesure le signal de FRET, on obtient un rapport signal/bruit bien meilleur, d'environ 520.

## Exemple 4

Dans cet exemple, la méthode selon l'invention a été utilisée pour quantifier l'expression des protéines EGFR et HER2, dans des échantillons de tumeurs issues de patients, en particulier des tumeurs mammaires.

La méthode a été mise en oeuvre de manière similaire à l'exemple 1 : les fluorophores Lumi4®Tb et d2 (Cisbio bioassays) ont été utilisés comme partenaires de FRET, respectivement donneur et accepteur. Pour la mesure de l'expression de EGFR, les anticorps cetuximab (Merck KGaA) et Ab-10 (Thermo Scientific), tous deux reconnaissant des épitopes distincts de EGFR, ont été utilisés et marqués respectivement par les fluorophores Lumi4®Tb et d2. Pour la quantification de HER2, les anticorps trastuzumab (Roche Pharma AG) et FRP5 (décrit par Harwerth IM et al (1992) J. Biol. Chem. 267: 15160-15167), tous deux spécifiques d'épitopes différents de HER2, ont été également conjugués avec Lumi4®Tb et d2 respectivement.

Pour chaque dosage, des cryosections de tumeur de 50 μm ont été incubées une nuit dans 180 μl de tampon TR-FRET (1X PBS / 10% BSA) contenant 50 nM de chacun des deux anticorps. Une coloration de l'ADN a ensuite été effectuée par ajout de 20 μl d'une solution de Hoechst 33342 (Invitrogen) à 0,1 mg/mL et incubation à température ambiante pendant 10 min. Après lavage et centrifugation, les échantillons ont été remis en suspension dans le tampon TR-FRET, soumis à une sonication et transférés dans une microplaque. Les signaux de fluorescence de Lumi4®Tb et d2 ont été mesurés respectivement à 620 et 665 nm en mode temps résolu (délai 60 μs ; fenêtre 400μs) après excitation à 337 nm en utilisant un fluorimètre Pherastar ® (BMG Labtech). Le signal du Hoechst 33342 a été mesuré en mode fluorescence à 460 nm. Ces signaux ont été corrigés par le bruit de fond selon la formule :

$$F_{corrigé} = F_{échantillon} - F_{bruitdefond},$$

dans laquelle les valeurs de $F_{bruitdefond}$ ont été obtenues en mesurant la fluorescence du tampon de TR-FRET seul.

Par ailleurs pour chaque dosage, les signaux de fluorescence mesurés sur des solutions obtenues par dilution en cascade au ½ (de façon à obtenir une gamme de concentration) à partir d'une solution mère contenant un mélange de 50 nM d'anticorps-Lumi4 ®-Tb et 50 nM d'anticorps - d2 ont été mesurés simultanément avec les échantillons, et le signal obtenu à 665 nm a été mis en relation avec celui mesuré à 620 nm pour chaque concentration d'anticorps. La courbe ainsi obtenue a été utilisée pour calculer la contribution de la fluorescence de Lumi4®-Tb à 665 nm ($F_{665Tb}$) à partir du signal émis par les échantillons à 620 nm . Le signal de TR-FRET a été exprimé de la manière suivante :

$$\Delta F_{665} = F_{665échantillon} - F_{665Tb}$$

Le signal du complexe ADN-Hoechst 33342 à 460 nm ($F_{460}$) a été utilisé pour normaliser le signal de TR-FRET pour tenir compte de la variabilité de la quantité de matériel biologique présent dans chaque milieu de mesure et en normalisant à une valeur moyenne de 100 000 unités de fluorescence (FU):

$TR\text{-}FRET_{normalisé} = (\Delta F_{665} \times 100\,000) / (F_{460})$. Le signal de TR-FRET normalisé a été exprimé en FU.

Afin de convertir le signal de TR-FRET normalisé en nombre de récepteurs par cellule, le nombre de récepteurs par cellule a été tout d'abord évalué sur des lignées cellulaires NIH/3T3 EGFR, NIH/3T3 HER2, NIH/3T3 EGFR/HER2 et SKOV-3. Pour cela, des cellules en culture ont été analysées par FACS en utilisant un dosage quantitatif indirect par immunofluorescence (QIFI kit, Dako) comme décrit précédemment (Gaborit et al. 2011 J Biol Chem., 286(13):11337-45). En parallèle, l'expression de EGFR et HER2 a été mesurée dans des échantillons de xénogreffes de souris dérivées des cellules tumorales correspondantes, en utilisant les essais TR-FRET. Ainsi, sur la base de l'hypothèse selon laquelle l'EGFR et HER2 sont exprimés à des niveaux comparables dans les cultures cellulaires et dans les xénogreffes de tumeurs dérivées de ces cellules, les valeurs obtenues dans les xénogreffes ont été utilisées comme étalons pour convertir le signal de TR-FRET en nombre de récepteurs par cellule.

*Résultats : Expression de EGFR et HER2*

Les résultats obtenus avec les 18 échantillons de tumeurs mammaires sont présentés dans la figure 4. Les niveaux médians d'expression observés sont de 2 800 EGFR / cellule (plage de 220 à 35 500) et de 49 800 HER2/cellule (plage de 11 500 à 584 000). Cinq des 18 tumeurs (soit 27,8%) exprimaient des niveaux très élevés de HER2 (216 800, 234 300, 391 000, 491 500 et 584 000 HER2/cellule). En moyenne, les niveaux d'expression de HER2 étaient 66 fois plus élevés que ceux d'EGFR. La reproductibilité des résultats a été vérifiée en reproduisant les expériences trois fois pour chaque échantillon. Les coefficients moyens de variation (CV) étaient de 22% pour EGFR et 19% pour l'analyse de quantification de HER2. Cette variabilité tient compte de la variabilité biologique, car des cryosections différentes ont été utilisées pour chaque expérience.

Pour confirmer la quantification de EGFR, l'ARN total de chaque tumeur a été extrait pour analyse RT-qPCR. Une corrélation linéaire positive (Rho= 0,84, P < 0,001) a été observée entre les niveaux d'expression de protéines EGFR déterminées par TR-FRET et les niveaux d'ARNm EGFR mesurés par RT-qPCR.

Pour valider les résultats de l'analyse de quantification de HER2 par la méthode selon l'invention, l'expression de HER2 a été évaluée en utilisant l'HercepTest™ ainsi qu'en mesurant l'amplification du gène codant pour HER2 par des analyses FISH et PCR quantitative. Les résultats de cette analyse, présentés dans la figure 5, n'ont montré aucun chevauchement dans les niveaux d'expression déterminés par TR-FRET entre les tumeurs HER2-Herceptest™ positives et HER2-Herceptest™ négatives. Seules les cinq tumeurs du sein avec > 150 000 HER2/cellule avaient un score HercepTest™ de 3+ et étaient positives pour les tests d'amplification génique de HER2. Cela indique que la méthode selon l'invention permet de détecter la surexpression de HER2 avec une spécificité et une sensibilité de 100% dans des échantillons tumoraux, ce qui la rend particulièrement précieuse pour évaluer la susceptibilité de patients à répondre à certains traitements anticancéreux visant HER2 (tels que le traitement par trastuzumab, Herceptin™).

Pour la première fois, une méthode fiable de quantification de HER2 pouvant être mise en oeuvre par exemple à l'hôpital, permet d'évaluer le nombre de protéines HER2 par cellule d'échantillons de patients. Elle ne présente pas les inconvénients de la coloration par immunohistochimie et de la technique FISH.

## Exemple 5

L'expression de HER2 a été quantifiée par la méthode décrite dans l'exemple 4 sur des échantillons congelés de tumeurs de 100 patients atteints d'un cancer du sein. La mesure de signaux de fluorescence normalisée a permis une mesure quantitative de l'expression des récepteurs HER2. La survie des patients sans maladie (« DFS » pour disease-free survival) et la survie globale (« OS » pour overall survival) ont été évaluées pour chaque patient. *Résultats*: Parmi les 100 patients, 82 étaient IHC-HER2 négatifs (HercepTest™ négatifs), dont 60 sujets qui étaient ER (récepteur de l'oestrogène) positifs et traités avec une thérapie hormonale. En utilisant des analyses de risques proportionnels de Cox, il a été montré que chez les sujets IHC-HER2 négatifs et ER positifs la présence de HER2 était significativement associée à la fois à une DFS (p = 0,0005) et une OS (p = 0,003) réduite.

La mesure quantitative de l'expression de HER2 par la méthode de l'invention peut permettre de prévoir l'issue de la maladie chez des sujets atteints de cancers du sein IHC-HER2 négatifs et ER positifs. Ce biomarqueur peut être utile pour identifier les patients dont le traitement hormonal n'est pas suffisamment efficace et qui pourraient bénéficier d'un traitement adjuvant par thérapie anti-HER de type Herceptin™. C'est là un des apports majeur de l'invention que de pouvoir améliorer la sélection des patients susceptibles de pouvoir répondre à ce type de traitement.

## Revendications

1. Méthode de quantification d'une protéine d'intérêt présente dans un échantillon de tissu solide, comprenant les étapes suivantes :

   (i) mise en contact d'un échantillon de tissu solide exprimant ladite protéine avec un premier ligand et un deuxième ligand, chacun de ces ligands étant capable de se lier de manière spécifique à un domaine de ladite protéine, et ces ligands étant respectivement marqués par un composé donneur et un composé accepteur, tous deux formant un couple de partenaires de FRET ;
   (ii) incubation de l'échantillon de tissu solide avec un agent de marquage fluorescent de l'ADN ;
   (iii) lavage de l'échantillon de tissu solide ;
   (iv) homogénéisation de l'échantillon de tissu solide sous forme de lysat cellulaire ;
   (v) mesure du signal de FRET émis par le milieu de mesure et normalisation de ce signal par le signal correspondant à la luminescence de l'agent de marquage fluorescent de l'ADN.

2. Méthode selon la revendication 1, **caractérisée en ce que** lesdits premier et deuxième ligands sont des anticorps.

3. Méthode selon la revendication 1, **caractérisée en ce que** le premier ligand est un ligand connu de la protéine d'intérêt et n'est pas un anticorps, et **en ce que** le deuxième ligand est un anticorps.

4. Méthode selon l'une quelconque des revendications 1 à 3, **caractérisée en ce que** la protéine d'intérêt est une protéine membranaire.

5. Méthode selon l'une quelconque des revendications 1 à 4, **caractérisée en ce que** la protéine d'intérêt est choisie parmi un récepteur membranaire couplé aux protéines G et un récepteur à activité tyrosine kinase.

6. Méthode selon l'une quelconque des revendications 1 à 5, **caractérisée en ce que** la protéine d'intérêt est choisie parmi EGFR, HER2, HER3 et HER4.

7. Méthode selon l'une quelconque des revendications 1 à 6, **caractérisée en ce que** le composé donneur est un chélate ou un cryptate de terre rare.

8. Méthode selon la revendication 7, caractérisée en que le composé donneur est un chélate ou un cryptate d'europium ou de terbium.

9. Méthode selon l'une quelconque des revendications 1 à 8, **caractérisée en ce que** le composé accepteur est choisi parmi les allophycocyanines, les rhodamines, les cyanines, les squaraines, les coumarines, les proflavines, les acridines, les fluorescéines, les dérivés du boron-dipyrrométhène, les fluorophores connus sous la dénomination « Atto », les fluorophores connus sous la dénomination «DY», les composés connus sous la dénomination « Alexa » et le nitrobenzoxadiazole.

10. Méthode selon l'une quelconque des revendications 1 à 9, **caractérisée en ce que** lesdits premier et second ligands sont introduits dans le milieu de mesure à une concentration finale supérieure à 10 nM.

11. Méthode selon l'une quelconque des revendications 1 à 10, **caractérisée en ce que** lesdits premier et second ligands sont introduits dans le milieu de mesure à une concentration finale comprise entre 20 et 80 nM.

12. Méthode selon l'une quelconque des revendications 1 à 11, **caractérisée en ce qu'**elle est mise en oeuvre sur un échantillon de tissu tumoral.

13. Méthode selon l'une quelconque des revendications 1 à 12, **caractérisée en ce que** lorsque le premier ou le deuxième ligand est un anticorps, l'épitope dudit anticorps est localisé sur un domaine de la protéine d'intérêt exposé au milieu extracellulaire.

14. Méthode selon l'une quelconque des revendications 1 à 13, **caractérisée en ce que** la protéine d'intérêt est la protéine HER2, et **en ce que** le premier ligand et le deuxième ligand sont des anticorps spécifiques de cette protéine.

15. Méthode *ex vivo* pour déterminer si un patient est éligible à un traitement thérapeutique par un anticorps se liant à la protéine HER2, **caractérisée en ce qu'**elle comprend la mise en oeuvre d'une méthode de quantification selon l'une quelconque des revendications 1 à 14 dans laquelle la protéine d'intérêt est la protéine HER2.

16. Méthode selon la revendication 15, **caractérisée en ce qu'**elle est mise en oeuvre sur un échantillon de tissu de patient dont les résultats d'analyse par immunohistochimie de l'expression de la protéine HER2 sont négatifs.

## Patentansprüche

1. Verfahren zur Quantifizierung eines in einer festen Gewebeprobe vorhandenen Proteins von Interesse, umfassend die folgenden Schritte:

   (i) Inkontaktbringen einer das Protein exprimierenden festen Gewebeprobe mit einem ersten Liganden und einem zweiten Liganden, wobei jeder dieser Liganden geeignet ist, an eine Domäne des Proteins spezifisch zu binden, und wobei diese Liganden jeweils durch eine Donor-Verbindung und eine Akzeptor-Verbindung markiert sind, wobei alle beide ein Paar von FRET-Partnern bilden,
   (ii) Inkubation der festen Gewebeprobe mit einem fluoreszierenden DNA-Markierungsmittel,
   (iii) Waschen der festen Gewebeprobe,
   (iv) Homogenisieren der festen Gewebeprobe in Form von Zelllysat,
   (v) Messen des durch das Messmedium ausgesandten FRET-Signals und Normalisieren dieses Signals durch das Signal, welches der Lumineszenz des fluoreszierenden DNA-Markierungsmittels entspricht.

2. Verfahren nach Anspruch 1, **dadurch gekennzeichnet, dass** der erste und der zweite Ligand Antikörper sind.

3. Verfahren nach Anspruch 1, **dadurch gekennzeichnet, dass** der erste Ligand ein bekannter Ligand des Proteins von Interesse und kein Antikörper ist und dass der zweite Ligand ein Antikörper ist.

**4.** Verfahren nach einem der Ansprüche 1 bis 3, **dadurch gekennzeichnet, dass** das Protein von Interesse ein Membranprotein ist.

**5.** Verfahren nach einem der Ansprüche 1 bis 4, **dadurch gekennzeichnet, dass** das Protein von Interesse aus einem G-Protein-gekoppelten Membranrezeptor und einem Rezeptor mit Tyrosinkinase-Aktivität ausgewählt ist.

**6.** Verfahren nach einem der Ansprüche 1 bis 5, **dadurch gekennzeichnet, dass** das Protein von Interesse aus EGFR, HER2, HER3 und HER4 ausgewählt ist.

**7.** Verfahren nach einem der Ansprüche 1 bis 6, **dadurch gekennzeichnet, dass** die Donor-Verbindung ein Chelat oder ein Cryptat von Seltene Erde ist.

**8.** Verfahren nach Anspruch 7, **dadurch gekennzeichnet, dass** die Donor-Verbindung ein Chelat oder ein Cryptat von Europium oder von Terbium ist.

**9.** Verfahren nach einem der Ansprüche 1 bis 8, **dadurch gekennzeichnet, dass** die Akzeptor-Verbindung aus den Allophycocyaninen, den Rhodaminen, den Cyaninen, den Squarainen, den Cumarinen, den Proflavinen, den Akridinen, den Fluoresceinen, den Derivaten von Boron-Dipyrromethen, den unter der Bezeichnung "Atto" bekannten Fluorophoren, den unter der Bezeichnung "DY" bekannten Fluorophoren, den unter der Bezeichnung "Alexa" bekannten Verbindungen und Nitrobenzoxadiazol ausgewählt ist.

**10.** Verfahren nach einem der Ansprüche 1 bis 9, **dadurch gekennzeichnet, dass** der erste und der zweite Ligand in einer Endkonzentration von mehr als 10 nM in das Messmedium eingebracht werden.

**11.** Verfahren nach einem der Ansprüche 1 bis 10, **dadurch gekennzeichnet, dass** der erste und der zweite Ligand in einer Endkonzentration im Bereich zwischen 20 und 80 nM in das Messmedium eingebracht werden.

**12.** Verfahren nach einem der Ansprüche 1 bis 11, **dadurch gekennzeichnet, dass** es an einer Tumorgewebeprobe durchgeführt wird.

**13.** Verfahren nach einem der Ansprüche 1 bis 12, **dadurch gekennzeichnet, dass** wenn der erste oder der zweite Ligand ein Antikörper ist, das Epitop des Antikörpers an einer zum extrazellulären Medium exponierten Domäne des Proteins von Interesse lokalisiert ist.

**14.** Verfahren nach einem der Ansprüche 1 bis 13, **dadurch gekennzeichnet, dass** das Protein von Interesse das HER2 Protein ist und dass der erste Ligand und der zweite Ligand spezifische Antikörper dieses Proteins sind.

**15.** *Ex vivo*-Verfahren zur Bestimmung, ob ein Patient für eine therapeutische Behandlung durch einen an das HER2 Protein bindenden Antikörper in Betracht kommt, **dadurch gekennzeichnet, dass** es die Durchführung eines Quantifizierungsverfahrens nach einem der Ansprüche 1 bis 14, bei dem das Protein von Interesse das HER2 Protein ist, umfasst.

**16.** Verfahren nach Anspruch 15, **dadurch gekennzeichnet, dass** es an einer Patientengewebeprobe durchgeführt wird, deren Ergebnisse einer Immunhistochemie-Analyse der Expression des HER2 Proteins negativ sind.

## Claims

**1.** A method for quantifying a protein of interest present in a solid tissue sample, comprising the following steps:

(i) bringing a solid tissue sample expressing said protein into contact with a first ligand and a second ligand, each of these ligands being capable of binding specifically to a domain of said protein, and these ligands being respectively labeled with a donor compound and an acceptor compound, both forming a pair of FRET partners;
(ii) incubating the solid tissue sample with an agent for fluorescently labeling of DNA;
(iii) washing the solid tissue sample;
(iv) homogenizing the solid tissue sample in the form of a cell lysate;
(v) measuring the FRET signal emitted by the measuring medium and normalizing this signal with the signal corresponding to the luminescence of the agent for fluorescently labeling of DNA.

2. The method as claimed in claim 1, **characterized in that** said first and second ligands are antibodies.

3. The method as claimed in claim, **characterized in that** the first ligand is a known ligand of the protein of interest and is not an antibody, and **in that** the second ligand is an antibody.

4. The method as claimed in any one claims 1 to 3, **characterized in that** the protein of interest is a membrane protein.

5. The method as claimed in any one of claims 1 to 4, **characterized in that** the protein of interest is chosen from a G-protein-coupled membrane receptor and a receptor tyrosine kinase.

6. The method as claimed in any one of claims 1 to 5, **characterized in that** the protein of interest is chosen from EGFR, HER2, HER3 and HER4.

7. The method as claimed in any one of claims 1 to 6, **characterized in that** the donor compound is a rare earth chelate or cryptate.

8. The method as claimed in claim 7, **characterized in that** the donor compound is a europium chelate or cryptate or a terbium chelate or cryptate.

9. The method as claimed in any one of claims 1 to 8, **characterized in that** the acceptor compound is chosen from allophycocyanins, rhodamines, cyanines, squaraines, coumarins, proflavins, acridines, fluoresceins, boron-dipyrromethene derivatives, fluorophores known under the name "Atto", fluorophores known under the name "DY", compounds known under the name "Alexa" and nitrobenzoxadiazole.

10. The method as claimed in any one of claims 1 to 9, **characterized in that** said first and second ligands are introduced into the measuring medium at a final concentration greater than 10 nM.

11. The method as claimed in any one of claims 1 to 10, **characterized in that** said first and second ligands are introduced into the measuring medium at a final concentration of between 20 and 80 nM.

12. The method as claimed in any one of claims 1 to 11, **characterized in that** it is carried out on a tumor tissue sample.

13. The method as claimed in any one of claims 1 to 12, **characterized in that**, when the first or the second ligand is an antibody, the epitope of said antibody is located on a domain of the protein of interest that is exposed to the extracellular medium.

14. The method as claimed in any one of claims 1 to 13, **characterized in that** the protein of interest is the HER2 protein, and **in that** the first and second ligands are antibodies specific for this protein.

15. An *ex vivo* method for determining whether a patient is eligible for a therapeutic treatment with an antibody which binds to the HER2 protein, **characterized in that** it comprises carrying out a method of quantification as claimed in any one of claims 1 to 14 in which the protein of interest is the HER2 protein.

16. The method as claimed in claim 15, **characterized in that** it is carried out on a tissue sample from a patient for whom the results of immunohistochemical analysis of the expression of the HER2 protein are negative.

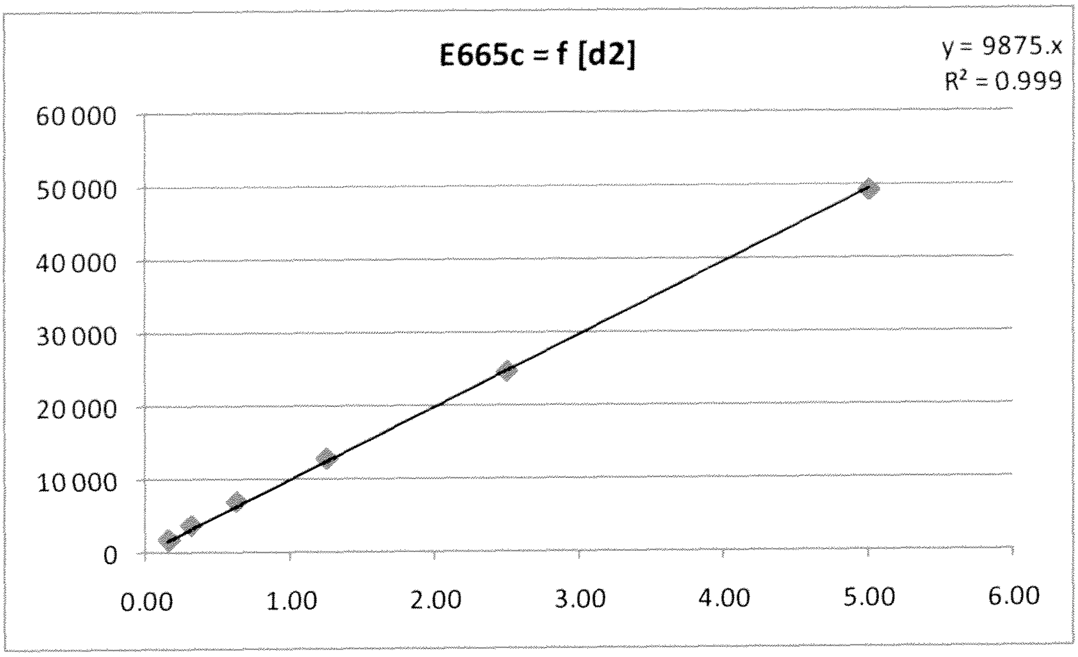
E665c = f [d2]
y = 9875.x
R² = 0.999
60 000
50 000
40 000
30 000
20 000
10 000
0
0.00
1.00
2.00
3.00
4.00
5.00
6.00

FIG.1

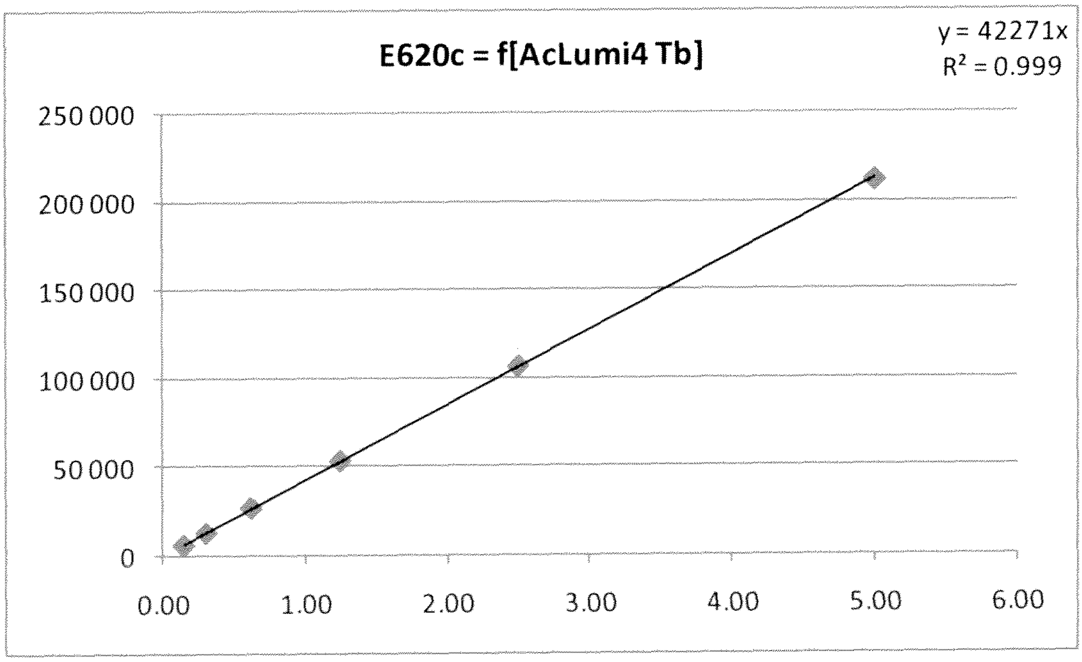
E620c = f[AcLumi4 Tb]
y = 42271x
R² = 0.999
250 000
200 000
150 000
100 000
50 000
0
0.00
1.00
2.00
3.00
4.00
5.00
6.00

FIG.2

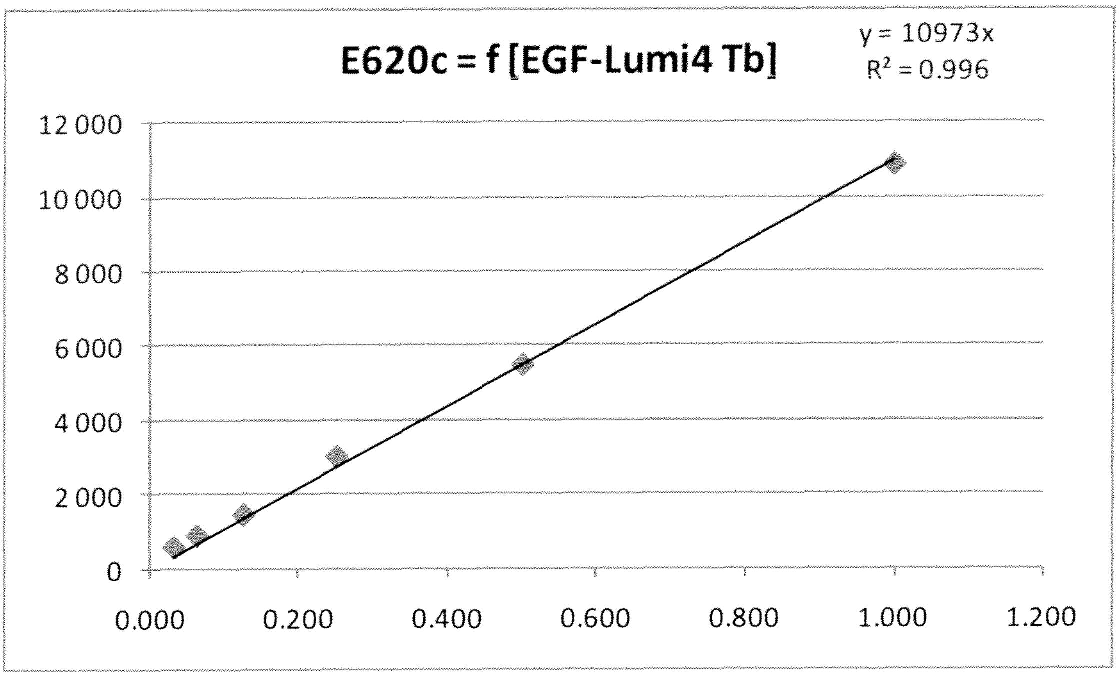
E620c = f [EGF-Lumi4 Tb]
y = 10973x
R² = 0.996
12 000
10 000
8 000
6 000
4 000
2 000
0
0.000
0.200
0.400
0.600
0.800
1.000
1.200

FIG.3

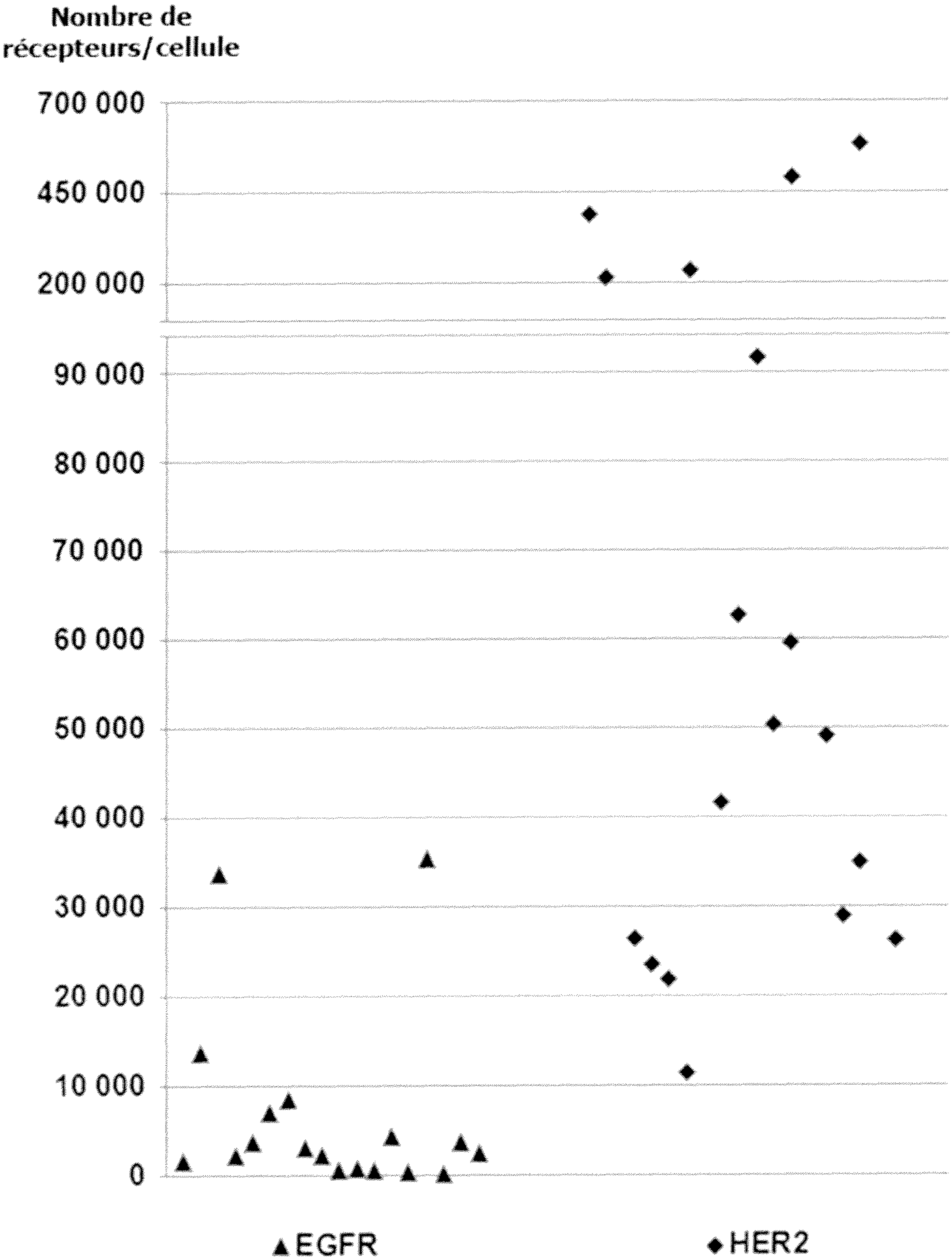
Nombre de
récepteurs/cellule
700 000
450 000
200 000
90 000
80 000
70 000
60 000
50 000
40 000
30 000
20 000
10 000
0
EGFR
HER2

FIG.4

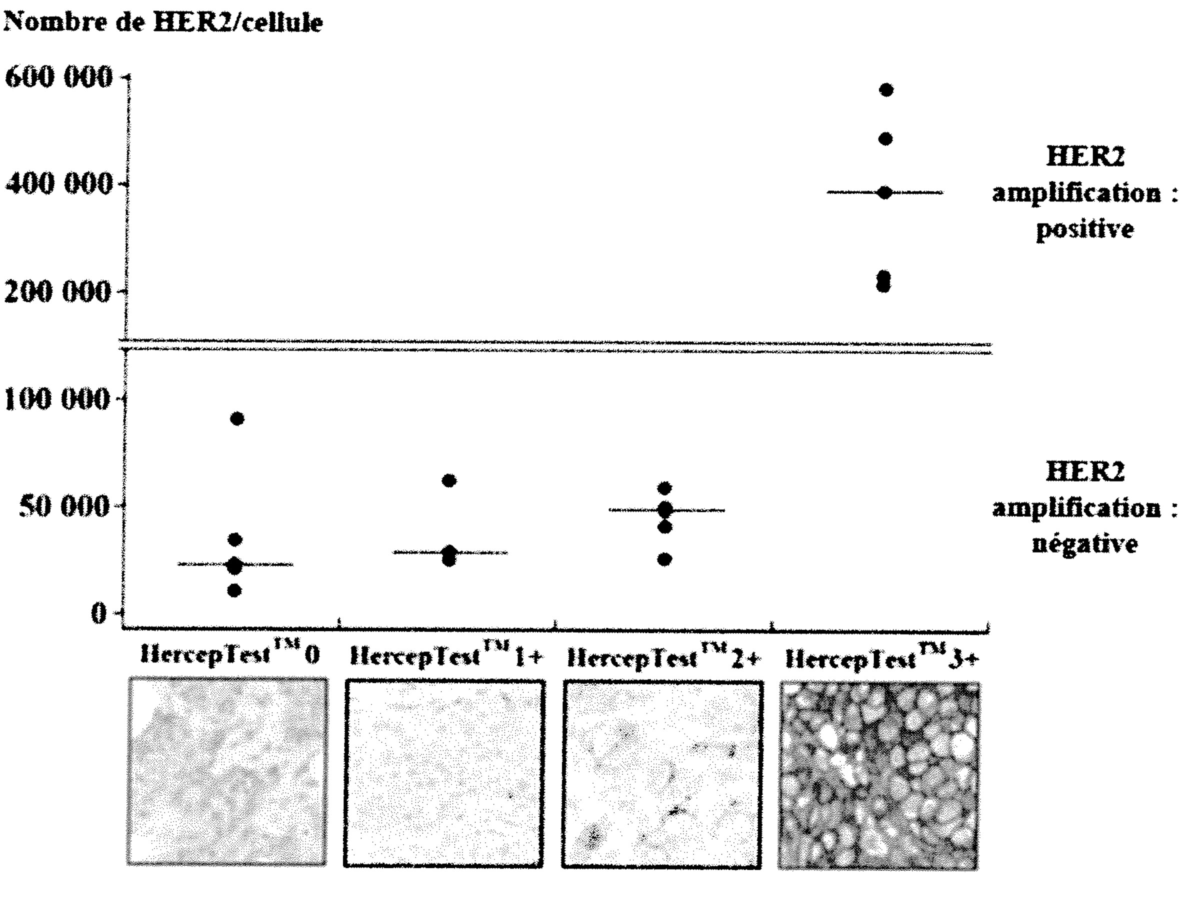
Nombre de HER2/cellule
600 000
400 000
200 000
100 000
50 000
0
HER2
amplification :
positive
HER2
amplification :
négative
HercepTest™ 0
HercepTest™ 1+
HercepTest™ 2+
HercepTest™ 3+

FIG.5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0180492 A
- EP 0321353 A
- EP 0601113 A
- WO 0196877 A
- US 4761481 A
- US 5032677 A
- US 5055578 A
- US 5106957 A
- US 5116989 A
- US 4801722 A
- US 4794191 A
- US 4637988 A
- US 4670572 A
- US 4837169 A
- US 4859777 A
- EP 0403593 A
- US 5324825 A
- US 5202423 A
- US 5316909 A
- WO 200910580 A
- EP 1154991 A
- EP 1154990 A
- WO 2008063721 A
- US 5622821 A
- FI 640680


### Littérature non-brevet citée dans la description


- **GABORIT et al.** *J. Biol. Chem.,* 2011, vol. 86 (13), 11337-11345
- **MAUREL et al.** *Analytical Biochemistry,* 2004, vol. 329 (2), 253-252
- **DIERMEIER-DAUCHER et al.** *Ann. N.Y. Acad. Sci.,* 2008, vol. 1130, 280-286
- **OKUNO et al.** GLIDA: GPCR ligand database for chemical genomics drug discovery database and tools update. *Nucl. Acids Res.,* vol. 36 (1), D907-912
- **C. KESSLER.** Nonisotopic probing, Blotting and Sequencing. Academic press Ltd, 1995, 66-72
- **JOSEPH R. LAKOWICZ.** Principles of fluorescence spectroscopy. Kluwer academic/plenum publishers, 1999
- **POOLE R. et al.** *Biomol. Chem,* 2005, vol. 3, 1013-1024
- **LATVA et al.** *Journal of Luminescence,* 1997, vol. 75, 149-169
- **SHINOBU.** *Molecular and Cellular Endocrinology,* 1984, vol. 37, 205
- **HARWERTH IM et al.** *J. Biol. Chem.,* 1992, vol. 267, 15160-15167
- **GABORIT et al.** *J Biol Chem.,* 2011, vol. 286 (13), 11337-45